# EUROPEAN PATENT APPLICATION

(11) **EP 3 251 517 A1**
(43) Date of publication of application: **06.12.2017**
(21) Application number: 16171948.9
(22) Date of filing: 30.05.2016
(51) Int. Cl.: A23D 7/00

(54) **NUTRITIONAL COMPOSITION**

(71) Applicant: Instituto Nacional de Medicina Genomica (INMEGEN), 14610 Mexico (MX)
(72) Inventor: TEJERO BARRERA, Elizabeth Maria, 03020 MEXICO (MX); BINIA, Aristea, 1700 Fribourg (CH); SILVA ZOLEZZI, Irma, 1084 Carrouge (CH); KUSSMANN, Martin, 1066 Epalinges (CH)
(74) Representative: Couzens, Patrick John

(57) **Abstract**

The present invention provides a nutritional composition comprising an omega-3 polyunsaturated fatty acid as an active agent for use in reducing insulin levels in a subject having a peroxisome proliferator-activated receptor-gamma 2 (PPARγ2) X12A genetic variant, where X is proline or alanine.

## Description

### FIELD OF THE INVENTION

The present invention relates to nutritional compositions and methods useful for reducing insulin levels in a subject.

### BACKGROUND TO THE INVENTION

Insulin is the pivotal hormone regulating cellular energy supply and macronutrient balance. It is secreted by pancreatic β cells and directs the anabolic processes of the fed state. Insulin is essential for the intracellular transport of glucose into insulin-dependent tissues such as muscle and adipose tissue (Wilcox; Clin Biochem Rev; 2005; pp19-39).

Insulin resistance is commonly defined where a normal or elevated insulin level produces an attenuated biological response (Cefalu; Exp Biol Med; 2001; 226; pp13-26); classically this refers to impaired sensitivity to insulin mediated glucose disposal (Reaven; Endocrinol Metab Clin North Am; 2004; 33; 283-303). Compensatory hyperinsulinaemia is a related state which occurs when pancreatic β cell secretion increases to maintain normal blood glucose levels in the setting of peripheral insulin resistance or reduced insulin sensitivity in muscle and adipose tissue. Increased fasting insulin levels, also known as hyperinsulinaemia, are indicative of reduced insulin sensitivity and may be a precursor to the development of conditions such as insulin resistance, pre-diabetes, metabolic syndrome, gestational diabetes and Type II Diabetes.

The effects of insulin, insulin sensitivity and insulin resistance vary according the physiological function of the tissues and organs concerned, and their dependence on insulin for metabolic processes. Tissues which are principally affected by reduced insulin sensitivity and potential insulin resistance include muscle, adipose tissue, liver, endothelium and vasculature, brain, pancreas, pituitary, kidney, gonads and bone (see Wilcox, as above). Factors which can influence insulin action and insulin resistance are wide ranging and include; diet and starvation, exercise and physical activity, stress, sleep and sleep deprivation, pregnancy and obesity.

The insulin resistance syndrome describes the cluster of abnormalities which occur more frequently in insulin resistant individuals. These include glucose intolerance, dyslipidemia, endothelial dysfunction and elevated procoagulant factors, haemodynamic changes, elevated inflammatory markers, abnormal uric acid metabolism, increased ovarian testosterone secretion and sleep-disordered breathing (see Reaven, as above). Clinical symptoms associated with insulin resistance include type 2 diabetes, cardiovascular disease, essential hypertension, polycystic ovary syndrome and non-alcoholic fatty liver disease (see Reaven, as above).

In view of the physiological effects and clinical conditions associated with reduced insulin sensitivity and insulin resistance, numerous studies have investigated potential methods for improving insulin sensitivity and preventing or reducing levels of insulin resistance.

The association between diet, fasting insulin levels and insulin resistance has encouraged investigations into how various dietary interventions affect insulin sensitivity. One intervention which has been investigated is omega-3 polyunsaturated fatty acids (omega-3 PUFA). However, the outcomes of these studies have been inconsistent and inconclusive.

By way of example, Ramel et al. reported that omega-3 PUFA supplementation improved insulin resistance in young, healthy individuals (Diabetolgia; 2008; 51; 1261-1268). Lopez-Alarcon et al. reported that omega-3 PUFA supplementation improved insulin resistance in children who were overweight and already clinically diagnosed as insulin resistant (Archives of Medical Research; 2011; 42; 502-508). In contrast, a meta-analysis published by the Cochrane Collaboration concluded that there was no significant change in fasting insulin levels following omega-3 PUFA supplementation (Hartweg et al.; 2008; Cochrane Database of Systematic Reviews, Issue 1. Art. No.: CD003205).

It is known that PUFAs, including omega-3 PUFA, are endogenous ligands for peroxisome proliferator-activated receptor (PPAR) γ (Kleiwer et al.; PNAS; 1997; 94; 4318-4323). Following ligand-induced activation, PPARγ heterodimerises with retinoid-X receptor α (RXRα) and can then bind to a peroxisome proliferator response element (PPRE) sequence located within a target gene promoter region, thereby initiating transcription. PPARγ regulates the expression of a number of genes involved in inflammation, adipose cell differentiation, atherosclerosis and metabolism (Debril et al.; J. Mol. Med; 2001; 79; 30-47).

A recent study in overweight individuals (average BMI of 26.5) showed that individuals carrying a PPARγ isoform 2 (PPARγ2) Pro12Ala variant - where the proline at position 12 of PPAR γ2 is substituted by an alanine residue - demonstrated a greater decrease in plasma triglyceride (TAG) levels following omega-3 PUFA supplementation than was observed for individuals homozygous for proline at position 12 of PPARγ2 (PPARγ2 P12P individuals) (Lindi et al., MGG 2003 79: 52-60). This study reported that the PPARγ2 Pro12Ala variant was not associated with changes in fasting concentrations of plasma glucose or serum insulin.

There remains a need for more robust and targeted nutritional approaches for reducing insulin levels *in vivo.*

### SUMMARY OF THE INVENTION

The present inventors have surprisingly found that individuals carrying a PPARγ2 X12A genetic variant, where X is proline or alanine, respond particularly well to omega-3 PUFA nutritional supplementation. In particular, individuals with a PPARγ2 X12A genetic allele had a greater reduction in fasting insulin levels following omega-3 PUFA nutritional supplementation compared to individuals homozygous for PPARγ2 Pro12 (i.e. PPARγ2 P12P).

Thus, in a first aspect the present invention provides an omega-3 PUFA or a nutritional composition comprising an omega-3 PUFA as an active agent for use in reducing insulin levels in a subject having a peroxisome proliferator-activated receptor-gamma 2 (PPARγ2) X12A genetic variant, where X is proline or alanine.

The subject may be suffering from a condition associated with elevated insulin levels.

In a further aspect, the present invention provides an omega-3 PUFA or a nutritional composition comprising an omega-3 PUFA as an active agent for use in treating or preventing reduced insulin sensitivity or a condition associated with reduced insulin sensitivity in a subject having a PPARγ2 X12A genetic variant, where X is a proline or alanine.

The condition associated with elevated insulin levels and/or reduced insulin sensitivity may be insulin resistance, prediabetes, type 2 diabetes, metabolic syndrome or cardiovascular disease.

The subject may, for example, be administered from about 500 to about 10000 mg omega-3 PUFA per day, preferably from about 1500 to about 3000 mg omega-3 PUFA per day.
In one embodiment, the omega-3 PUFA is eicosapentanoic acid (EPA) and/or docosahexanoic acid (DHA) or a mixture of EPA and DHA.

The omega-3 PUFA may comprise an EPA to DHA ratio of about 0.5:1 to about 5:1, for example 0.5:1, 1:1, 1.5:1, 2:1, 2.5:1, 3:1 or 5:1 wt/wt; preferably about 2.5:1 wt/wt.

The subject may be administered, for example, from about 500 to about 4000 mg EPA and from about 500 to about 4000 mg DHA per day, preferably from about 1000 to about 3000 mg EPA and from about 500 to about 1000 mg DHA per day.

The nutritional composition may be in the form of a food stuff, for example a human food stuff. The nutritional composition may be in the form of a complete nutritional product.

The nutritional composition may be in the form of a capsule, a tablet, a powdered form, a freeze-dried product or an oil-in-water emulsion.

In a further aspect the present invention relates to a method for reducing insulin levels in a subject comprising administering an omega-3 PUFA or a nutritional composition comprising an omega-3 PUFA as an active agent to the subject; wherein the subject has been identified as having a PPARγ2 X12A genetic variant, where X is a proline or alanine.

In another aspect the present invention relates to a method for reducing insulin levels in a subject comprising the steps of:
i) determining whether the subject has a PPARγ2 X12A genetic variant; and
ii) administering an omega-3 PUFA or a nutritional composition comprising an omega-3 PUFA as an active agent to a subject identified has having a PPARγ2 X12A genetic variant; where X is a proline or alanine.

The present methods may be for use in treating or preventing reduced insulin sensitivity or a disease associated therewith, insulin resistance, prediabetes, type 2 diabetes, metabolic syndrome, gestational diabetes or cardiovascular disease.

In a further aspect the present invention relates to the use of an omega-3 PUFA in the preparation of a nutritional composition for reducing insulin levels in a subject, wherein the subject has a PPARγ2 X12A genetic variant; where X is a proline or alanine.

### DESCRIPTION OF THE FIGURES

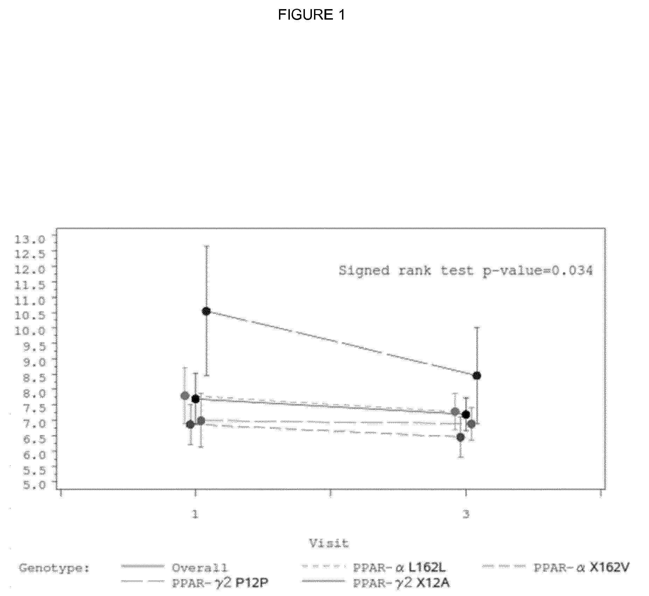

**Figure 1** **-** Fasting insulin levels before and after a six week supplementation with omega-3 PUFA

### DETAILED DESCRIPTION OF THE INVENTION

### INSULIN

The nutritional compositions comprising an omega-3 PUFA described herein are used to reduce insulin levels in a subject with a PPARγ2 X12A variant, where X is proline or alanine.

Insulin is an anabolic hormone secreted by the pancreatic β cells. It promotes glucose uptake, glycogenesis, lipogenesis, and protein synthesis of skeletal muscle and fat tissue through the tyrosine kinase receptor pathway. In addition, insulin is the most important factor in the regulation of plasma glucose homeostasis, as it counteracts glucagon and other catabolic hormones such as epinephrine, glucocorticoid, and growth hormone.

Insulin secretion can be divided into basal (postabsorptive) and stimulated (postprandial) states. The former prevails during the interprandial phases and plays a major role during the overnight fast; the latter regulates glucose metabolism when carbohydrate is abundant and must be disposed of.

Insulin sensitivity refers to an individual's ability to respond to insulin. In the fasting state, the basal insulin secretory rate increases as a function of the progressive decline in insulin action. As such, the fasting insulin concentration is often taken as a marker for insulin sensitivity.

Low levels of fasting insulin are typically associated with good health. High fasting insulin levels may be indicative of reduced insulin sensitivity and/or inappropriate insulin secretion control by pancreatic β-cells.

Such high fasting insulin levels may indicate that the subject is at greater risk of developing conditions which are associated with reduced insulin sensitivity.

The insulin levels may be fasting insulin levels or postprandial insulin levels.

The term "reducing" is synonymous with terms such as decreasing and lowering. The term "levels" refers to any measure of abundance such as amount or concentration. Thus, the present compositions and methods are useful for decreasing insulin levels in a subject.

Reducing insulin levels may mean that insulin levels are reduced by at least 5%, at least 7%, at least 10%, at least 20%, at least 30%, at least 40%,at least 50%, at least 60%, or at least 70% compared to the insulin level prior to the administration of a nutritional composition comprising an omega-3 PUFA.

In one embodiment, the insulin levels are fasting insulin levels.

Fasting insulin levels are typically expressed as milli-international-units per litre (mIU/L) or pmol/L. Typically a subject is instructed to fast for at least 8 hours before plasma or serum insulin levels are determined. Levels of insulin may be determined using methods which are well known in the art, for example an enzyme-linked immunosorbent assay (ELISA). In one embodiment insulin levels are determined by measuring fasting insulin levels. In one embodiment fasting insulin levels may be reduced by at least 5%, at least 7%, at least 10%, at least 20%, at least 30%, at least 40%, or at least 50% compared to the fasting insulin level prior to the administration of a nutritional composition comprising an omega-3 PUFA.

Insulin levels are typically determined in blood serum or plasma. Techniques for collecting blood samples and separating blood fractions are well known in the art. For instance, vena blood samples can be collected from patients using a needle and deposited into plastic tubes. The collection tubes may, for example, contain spray-coated silica and a polymer gel for serum separation. Serum can be separated by centrifugation at 1300 RCF for 10 min at room temperature and stored in small plastic tubes at -80°C.

In one embodiment, the present invention relates to an omega-3 PUFA or a nutritional composition comprising an omega-3 PUFA for use in reducing insulin levels in a subject suffering from elevated insulin levels. Such conditions include, for example, insulin resistance, prediabetes, type 2 diabetes, metabolic syndrome or cardiovascular disease.

In one embodiment, the present invention relates to an omega-3 PUFA or a nutritional composition as described herein for use in the prevention and/or treatment of a condition associated with elevated insulin levels and/or reduced insulin sensitivity.

The prevention of a condition associated with reduced insulin sensitivity relates to the prophylactic use of an omega-3 PUFA or a nutritional composition as described herein. Accordingly, the nutritional composition may be administered to a subject who has not yet developed a condition and/or who is not showing any symptoms of the condition to prevent or impair the cause of condition or to reduce or prevent development of at least one symptom associated with the condition.

### REDUCED INSULIN SENSITIVITY

Conditions associated with reduced insulin sensitivity include insulin resistance, prediabetes, type 2 diabetes, metabolic syndrome, gestational diabetes or cardiovascular disease.

Insulin resistance is a condition in which the body produces insulin but does not use it effectively. When people have insulin resistance, glucose builds up in the blood instead of being absorbed by the cells, leading to type 2 diabetes or prediabetes. Insulin resistance may be defined as a reduced responsiveness of a target cell or a whole organism to the insulin concentration to which it is exposed.

Prediabetes is the medical stage in which not all of the symptoms required to diagnose a person as diabetic are present, but blood sugar is abnormally high. Prediabetes usually occurs in people who already have insulin resistance. Although insulin resistance alone does not cause type 2 diabetes, it often sets the stage for the disease by placing a high demand on the insulin-producing beta cells. In prediabetes, the beta cells can no longer produce enough insulin to overcome insulin resistance, causing blood glucose levels to rise above the normal range.

Type 2 diabetes is a chronic metabolic disorder which is increasing in prevalence globally. In some countries of the world the number of people affected is expected to double in the next decade due to an increase in the ageing population.

Type 2 diabetes is characterized by insulin insensitivity as a result of insulin resistance, declining insulin production, and eventual pancreatic beta-cell failure. This leads to a decrease in glucose transport into the liver, muscle cells, and fat cells. There is an increase in the breakdown of fat associated with hyperglycemia.

As a result of this dysfunction, glucagon and hepatic glucose levels that rise during fasting are not suppressed with a meal. Given inadequate levels of insulin and increased insulin resistance, hyperglycemia results.

People with type 2 diabetes are more vulnerable to various forms of both short- and long-term complications, including diabetic ketoacidosis (DKA), hyperosmolar hyperglycaemic state (HHS), retinopathy, cardiopathy, nephropathy and neuropathy. These complications may lead to premature death.

Prediabetes and diabetes may be diagnosed by the HbA1c test, which is a blood test that reflects the average of a person's blood glucose levels over the past 3 months and does not show daily fluctuations. Glucose levels determined using Hb1Ac tests are typically expressed as SI units (i.e., HbA1c/total Hb (mmol/mol)) and/or derived NGSP/DCCT units (%). The master equations for converting IFCC units into NGSP units (NGSP%=0.0915xIFCC mmol/mol+2.15) and vice versa (IFCC mmol/mol=10.93 NGSP%-23.5) are established and monitored by the IFCC and NGSP networks (Weykamp et al.; Clin Chem. 2008;54:240-248). The higher the percentage, the higher a person's blood glucose levels have been. A normal HbA1c level is typically below 5.7 percent. Prediabetes and diabetes may also be diagnosed using a traditional fasting plasma glucose (FPG) test or oral glucose tolerance test (OGTT).

The correlation of each of these tests with prediabetes and diabetes is shown in Table 2.

**Table 2**

| | **Hb1Ac (percent)** | **Fasting Plasma Glucose (mg/dL)** | **Oral Glucose Tolerance Test (mg/dL)** |
|---|---|---|---|
| **Diabetes** | 6.5 or above | 126 or above | 200 or above |
| **Prediabetes** | 5.7 to 6.4 | 100 to 125 | 140 to 199 |
| **Normal** | About 5 | 99 or below | 139 or below |

Metabolic syndrome is a clustering of at least three of five of the following medical conditions: abdominal (central) obesity, elevated blood pressure, elevated fasting plasma glucose, high serum triglycerides, and low high-density lipoprotein (HDL) levels. Metabolic syndrome is associated with the risk of developing cardiovascular disease and diabetes.

Gestational diabetes (GDM) is a condition in which women without previously diagnosed diabetes exhibit high blood glucose levels during pregnancy (especially during their third trimester). Gestational diabetes may be caused by altered insulin receptor function caused by pregnancy-related factors. For example, human placental lactogen may interfere with susceptible insulin receptors and cause inappropriately elevated blood sugar levels. Women with unmanaged gestational diabetes are at increased risk of developing type 2 diabetes after pregnancy, as well as having a higher incidence of pre-eclampsia and Caesarean section. Moreover, their offspring are prone to developing childhood obesity, with type 2 diabetes later in life.

Cardiovascular disease (CVD) is a class of diseases that involve the heart or blood vessels. Cardiovascular disease includes coronary artery diseases (CAD) such as angina and myocardial infarction (commonly known as a heart attack). Other CVDs are stroke, hypertensive heart disease, rheumatic heart disease, cardiomyopathy, atrial fibrillation, congenital heart disease, endocarditis, aortic aneurysms, peripheral artery disease and venous thrombosis.

### PPARγ2

Peroxisome proliferator-activated receptor γ (PPARγ) is a member of the nuclear hormone receptor superfamily. It is known to be involved in regulating the expression of genes related to inflammation, adipose cell differentiation, atherosclerosis and metabolism. The major ligands for PPARγ are PUFA and prostanoids. Following ligand-induced activation, PPARγ heterodimerises with retinoid-X receptor α (RXRα) and can then bind to a peroxisome proliferator response element (PPRE) sequence located within a target gene promoter region, thereby initiating transcription.

Four subtypes of PPARγ mRNA transcribed from different promoters give rise to two different PPARγ proteins. The PPARγ2 protein is exclusively expressed in adipose tissue. An example human PPARγ2 protein is the human PPARγ2 protein having the UniProtKB accession number P37231. This exemplified sequence is 505 amino acids in length and is shown as SEQ ID NO: 1.
SEQ ID NO: 1 (PPARγ2 amino acid sequence)

The most widely studied variant in PPARγ2 is the Pro12Ala variant in which the proline at position 12 is substituted by an alanine residue (this corresponds to position 12 of SEQ ID NO: 1). This mutation is caused by the rs1801282 single nucleotide polymorphism (SNP) at codon 12 of PPARγ2-specific exon B (Pro12Ala, CCA→GCA).

According to the present invention, the subject has a PPARγ2 X12A genetic variant, where X is proline or alanine. Thus, X is a proline or alanine residue encoded by a first PPARγ2 allele and A is an alanine residue encoded by a second PPARγ2 allele. Accordingly, the subject may be homozygous or heterozygous for the alanine residue at position 12 of PPARγ2 and may therefore have a PPARγ2 P12A or A12A genotype.

### DETECTING GENETIC VARIANTS

Genetic variants may be detected using a wide range of techniques and methods which are well known in the art.

A PPARγ2 genetic variant may be detected by detecting the SNP associated with the PPARγ2 Pro12Ala variant.

### Detection of alleles

Nucleic acids obtained from a sample can be genotyped to identify the particular allele present for a marker locus. A sample of sufficient quantity to permit direct detection of marker alleles from the sample may be obtained.

Alternatively, a smaller sample is obtained from the subject and the nucleic acids are amplified prior to detection. Optionally, the nucleic acid sample is purified (or partially purified) prior to detection of the marker alleles.

Examples of allele detection methods are given below:

### Allele Specific PCR

Allele-specific PCR differentiates between target regions differing in the presence of absence of a variation or polymorphism. PCR amplification primers are chosen based upon their complementarity to the target sequence, such as a sequence disclosed herein. The primers bind only to certain alleles of the target sequence.

### Allele Specific Oligonucleotide Screening Methods

Further screening methods employ the allele-specific oligonucleotide (ASO) screening methods (e.g. see Saiki et al., Nature 324:163-166, 1986).

Oligonucleotides with one or more base pair mismatches are generated for any particular allele. ASO screening methods detect mismatches between one allele in the target genomic or PCR amplified DNA and the other allele, showing decreased binding of the oligonucleotide relative to the second allele (i.e. the other allele) oligonucleotide. Oligonucleotide probes can be designed that under low stringency will bind to both polymorphic forms of the allele, but which at high stringency, bind to the allele to which they correspond. Alternatively, stringency conditions can be devised in which an essentially binary response is obtained, i.e., an ASO corresponding to a variant form of the target gene will hybridize to that allele, and not to the wildtype allele.

### Ligase Mediated Allele Detection Method

Ligase can also be used to detect point mutations, such as the SNPs in a ligation amplification reaction (e.g. as described in Wu et al., Genomics 4:560-569, 1989). The ligation amplification reaction (LAR) utilizes amplification of specific DNA sequence using sequential rounds of template dependent ligation (e.g. as described in Wu, supra, and Barany, Proc. Nat. Acad. Sci. 88:189-193, 1990).

### Denaturing Gradient Gel Electrophoresis

Amplification products generated using the polymerase chain reaction can be analyzed by the use of denaturing gradient gel electrophoresis. Different alleles can be identified based on the different sequence-dependent melting properties and electrophoretic migration of DNA in solution. DNA molecules melt in segments, termed melting domains, under conditions of increased temperature or denaturation.

Each melting domain melts cooperatively at a distinct, base-specific melting temperature (Tm). Melting domains are at least 20 base pairs in length, and can be up to several hundred base pairs in length.

Differentiation between alleles based on sequence specific melting domain differences can be assessed using polyacrylamide gel electrophoresis, as described in Chapter 7 of Erlich, ed., PCR Technology, Principles and Applications for DNA Amplification, W. H. Freeman and Co., New York (1992).

Generally, a target region to be analyzed by denaturing gradient gel electrophoresis is amplified using PCR primers flanking the target region. The amplified PCR product is applied to a polyacrylamide gel with a linear denaturing gradient as described in Myers et al., Meth. Enzymol. 155:501-527, 1986, and Myers et al., in Genomic Analysis, A Practical Approach, K. Davies Ed. IRL Press Limited, Oxford, pp. 95 139, 1988. The electrophoresis system is maintained at a temperature slightly below the Tm of the melting domains of the target sequences.

In an alternative method of denaturing gradient gel electrophoresis, the target sequences can be initially attached to a stretch of GC nucleotides, termed a GC clamp, as described in Chapter 7 of Erlich, supra. In one example, at least 80% of the nucleotides in the GC clamp are either guanine or cytosine. In another example, the GC clamp is at least 30 bases long. This method is particularly suited to target sequences with high Tm's.

Generally, the target region is amplified by the polymerase chain reaction as described above. One of the oligonucleotide PCR primers carries at its 5' end, the GC clamp region, at least 30 bases of the GC rich sequence, which is incorporated into the 5' end of the target region during amplification. The resulting amplified target region is run on an electrophoresis gel under denaturing gradient conditions as described above. DNA fragments differing by a single base change will migrate through the gel to different positions, which can be visualized by ethidium bromide staining.

### Temperature Gradient Gel Electrophoresis

Temperature gradient gel electrophoresis (TGGE) is based on the same underlying principles as denaturing gradient gel electrophoresis, except the denaturing gradient is produced by differences in temperature instead of differences in the concentration of a chemical denaturant. Standard TGGE utilizes an electrophoresis apparatus with a temperature gradient running along the electrophoresis path. As samples migrate through a gel with a uniform concentration of a chemical denaturant, they encounter increasing temperatures. An alternative method of TGGE, temporal temperature gradient gel electrophoresis (TTGE or tTGGE) uses a steadily increasing temperature of the entire electrophoresis gel to achieve the same result. As the samples migrate through the gel the temperature of the entire gel increases, leading the samples to encounter increasing temperature as they migrate through the gel. Preparation of samples, including PCR amplification with incorporation of a GC clamp, and visualization of products are the same as for denaturing gradient gel electrophoresis.

### Single-Strand Conformation Polymorphism Analysis

Target sequences or alleles can be differentiated using single-strand conformation polymorphism analysis, which identifies base differences by alteration in electrophoretic migration of single stranded PCR products, for example as described in Orita et al., Proc. Nat. Acad. Sci. 85:2766-2770, 1989. Amplified PCR products can be generated as described above, and heated or otherwise denatured, to form single stranded amplification products. Single-stranded nucleic acids can refold or form secondary structures which are partially dependent on the base sequence. Thus, electrophoretic mobility of single-stranded amplification products can detect base-sequence difference between alleles or target sequences.

### Chemical or Enzymatic Cleavage of Mismatches

Differences between target sequences can also be detected by differential chemical cleavage of mismatched base pairs, for example as described in Grompe et al., Am. J. Hum. Genet. 48:212-222, 1991. In another method, differences between target sequences can be detected by enzymatic cleavage of mismatched base pairs, as described in Nelson et al., Nature Genetics 4:11-18, 1993. Briefly, genetic material from an animal and an affected family member can be used to generate mismatch free heterohybrid DNA duplexes. As used herein, 'heterohybrid' means a DNA duplex strand comprising one strand of DNA from one animal, and a second DNA strand from another animal, usually an animal differing in the phenotype for the trait of interest.

### Non-gel Systems

Other possible techniques include non-gel systems such as TaqMan™ (Perkin Elmer). In this system oligonucleotide PCR primers are designed that flank the mutation in question and allow PCR amplification of the region. A third oligonucleotide probe is then designed to hybridize to the region containing the base subject to change between different alleles of the gene. This probe is labeled with fluorescent dyes at both the 5' and 3' ends. These dyes are chosen such that while in this proximity to each other the fluorescence of one of them is quenched by the other and cannot be detected. Extension by Taq DNA polymerase from the PCR primer positioned 5' on the template relative to the probe leads to the cleavage of the dye attached to the 5' end of the annealed probe through the 5' nuclease activity of the Taq DNA polymerase. This removes the quenching effect allowing detection of the fluorescence from the dye at the 3' end of the probe. The discrimination between different DNA sequences arises through the fact that if the hybridization of the probe to the template molecule is not complete, i.e. there is a mismatch of some form, the cleavage of the dye does not take place. Thus only if the nucleotide sequence of the oligonucleotide probe is completely complimentary to the template molecule to which it is bound will quenching be removed. A reaction mix can contain two different probe sequences each designed against different alleles that might be present thus allowing the detection of both alleles in one reaction.

Many current methods for the detection of allelic variation are reviewed by Nollau et ah, Clin. Chem. 43, 1114-1120, 1997; and in standard textbooks, for example "Laboratory Protocols for Mutation Detection", Ed. by U. Landegren, Oxford University Press, 1996 and "PCR", 2nd Edition by Newton & Graham, BIOS Scientific Publishers Limited, 1997.

### SAMPLE

The test sample of nucleic acid is conveniently a sample of blood, mouth swab, biopsy, or other body fluid or tissue obtained from an individual. It will be appreciated that the test sample may equally be a nucleic acid sequence corresponding to the sequence in the test sample, that is to say that all or a part of the region in the sample nucleic acid may firstly be amplified using any convenient technique e.g. PCR, before analysis of allelic variation. The invention includes the detection of the polymorphism determined from a nucleic acid sample (which may be as defined above) that has already been removed from the individual.

### OMEGA-3 POLYUNSATURATED FATTY ACID

Polyunsaturated fatty acids (PUFA) can be classified into two major families (depending on the position (n) of the first double bond nearest the methyl end of the fatty acid carbon chain). Thus, the omega-6 fatty acids have the first unsaturated double bond six carbon atoms from the omega (methyl) end of the molecule and additionally have a total of two or more double bonds, with each subsequent unsaturation occurring 3 additional carbon atoms toward the carboxyl end of the molecule. In contrast, the omega-3 fatty acids have the first unsaturated double bond three carbon atoms away from the omega end of the molecule and additionally have a total of three or more double bonds, with each subsequent unsaturation occurring 3 additional carbon atoms toward the carboxyl end of the molecule.

Table 3 summarizes the common names of omega-3 fatty acids and the abbreviations that will be used throughout the specification:

**Table 3**

| Common Name | Abbreviation | Shorthand notation |
|---|---|---|
| oleic acid | OA | 18:1^{Δ9} |
| Linoleic acid | LA | 18:2^{Δ9,12} |
| γ-Linolenic | GLA | 18:3^{Δ6,9,12} |
| di-homo γ-linolenic acid | DGLA | 20:3^{Δ8,11,14} |
| Arachidonic acid | ARA | 20:4^{Δ5,8,11,14} |
| α-linolenic acid | ALA | 18:3^{Δ9,12,15} |
| stearidonic acid | SDA | 18:4^{Δ6,9,12,15} |
| eicosatetraenoic acid | ETA | 20:4^{Δ8,11,14,17} |
| eicosapentaenoic acid | EPA | 20:5^{Δ5,8,11,14,17} |
| docosapentaenoic acid | DPA | 22:5^{Δ7,10,13,16,19} |
| docosahexaenoic acid | DHA | 22:6^{Δ4,7,10,13,16,19} |

The three main types of omega-3 PUFA involved in human physiology are α-linolenic acid (ALA) (found in plant oils), eicosapentaenoic acid (EPA), and docosahexaenoic acid (DHA) (both commonly found in marine oils). Currently the primary dietary source of omega-3 highly unsaturated fatty acids is from certain fish oils which can contain up to 20-30% of these fatty acids in their triacylglycerides. Consequently large quantities of fish oil are processed and encapsulated each year for sale as a dietary supplement. Common sources of plant oils containing ALA include walnut, edible seeds, clary sage seed oil, algal oil, flaxseed oil, Sacha Inchi oil, Echium oil, and hemp oil. Sources of EPA and DHA include fish oils, egg oil, squid oils, and krill oil.

The omega-3 PUFA may be provided in small amounts of oils containing high quantities of omega-3 PUFA, such as fish oils or microbial oils.

The omega-3 PUFA is preferably selected from EPA (C20:5, omega-3) and DHA (C22:6, omega-3) or a mixture of EPA and DHA.

The omega-3 PUFA, for example as provided in a nutritional composition as described herein, may comprise an EPA to DHA ratio of about 0.5:1 to 5:1, 1:1 to 3:1 or 1.5:1 to 2.5:1 wt/wt. The omega-3 PUFA may comprise an EPA to DHA ratio of about 0.5:1, 1:1, 1.5:1, 2:1, 2.5:1, 3:1 or 5:1 wt/wt. Preferably, the omega-3 PUFA comprises an EPA to DHA ratio of about 2.5:1 wt/wt.

An example of a commercial source of omega-3 PUFA is GNC Preventive Nutrition® Triple Strength Fish Oil.

### DOSAGE

The actual dosage of omega-3 PUFA may be varied to provide a dose which will be most suitable for an individual subject. The dose may vary with the age, weight and response of the particular subject.

The subject may, for example, be administered from about 500 to about 10000 mg omega-3 PUFA per day. In one embodiment the subject is administered from about 1500 to about 3000 mg omega-3 PUFA per day.

The subject may, for example, be administered about 500 to 4000 mg EPA per day. In one embodiment the subject is administered about 1000 to 3000 mg EPA per day. In one embodiment the subject is administered about 2000 mg EPA per day.

The subject may, for example, be administered about 500 to 4000 mg DHA per day. In one embodiment the subject is administered about 500 to 3000, 500 to 2000 or 500 to 1000 mg DHA per day. In one embodiment the subject is administered about 500 to 1000 mg DHA per day. In one embodiment the subject is administered about 750 mg DHA per day.

The subject may, for example, be administered about 500 to 4000 mg EPA and about 500 to 4000 mg DHA per day. In one embodiment the subject is administered about 1000 to 3000 mg EPA and about 500 to 1000 mg DHA per day. In one embodiment the subject is administered about 2000 mg EPA and about 750 mg DHA per day.

### NUTRITIONAL COMPOSITION

The term nutritional composition means a composition which nourishes a subject. The nutritional composition is usually to be taken orally, intragastrically or intravenously. Preferably, the nutritional composition for use in the present invention is to be taken orally.

Nutritional compositions, as used herein, may include any number of optional ingredients in addition to omega-3 PUFA. Such additional ingredients include, but are not limited to, conventional food additives (synthetic or natural), for example one or more acidulants, additional thickeners, buffers or agents for pH adjustment, chelating agents, colorants, emulsifies, excipient, flavor agent, mineral, osmotic agents, a pharmaceutically acceptable carrier, preservatives, stabilizers, sugar, sweeteners, texturizers, and/or vitamins. The optional ingredients can be added in any suitable amount.

The nutritional composition may be in the form of powder, tablets, capsules, pastilles or a liquid for example. The composition may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The composition may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

The composition may be usable for reconstitution in water. The composition may be an oil-in-water emulsion.

The nutritional composition may contain a carbohydrate source in addition to the omega-3 PUFA.

In one embodiment, at least 70, 80, 90, 95, 98, 99 or 100% of the PUFA in the nutritional composition are omega-3 PUFA. In one embodiment, the nutritional composition does not comprise omega-6 PUFA.

The nutritional composition may contain vitamins and minerals understood to be essential in the daily diet and in nutritionally significant amounts. Minimum requirements have been established for certain vitamins and minerals. Examples of minerals, vitamins and other nutrients optionally present in the composition include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L- carnitine. Minerals are usually added in salt form. The presence and amounts of specific minerals and other vitamins will vary depending on the intended population.

The nutritional composition may also contain other substances which may have a beneficial effect such as lactoferrin, nucleotides, nucleosides, gangliosides, polyamines, phytosterols, fibres, lipids and the like.

The nutritional composition may be in the form of a nutritional supplement. A nutritional supplement refers to a product which is intended to supplement the general diet of a subject.

The composition may be in the form of a complete nutritional product. A complete nutritional product refers to a product which is intended to be the sole item or meal or diet consumed by a subject. As such, a complete nutritional product may contain sufficient types and levels of macronutrients (proteins, fats and carbohydrates) to be sufficient to be a sole source of nutrition for the subject to which it is being administered.

The composition may be inserted or mixed into a food substance. The composition may be in the form of a food stuff, for example a human food stuff.

The nutritional composition may be in the form of a tablet or capsule.

The nutritional composition may be enriched with omega-3 PUFA. 'Enriched' means that omega-3 PUFA has been added to the composition. For example, omega-3 PUFA may be spiked (i.e. added within or into) the composition.

In one embodiment, where a nutritional composition natively contains omega-3 PUFA, enriched with omega-3 PUFA means that the enriched composition comprises a greater amount of the compound than occurs naturally natively in the composition.

For example, an enriched composition may comprise at least 1.5-, at least 2-, at least 5-, at least 10-, at least 20-, at least 50- or at least 100-fold more omega-3 PUFA than an equivalent naturally occurring native composition which has not been enriched.

Enrichment may be achieved by adding omega-3 PUFA to the composition, for example by spraying or spiking it with omega-3 PUFA.

### SUBJECT

The subject may be, but is not limited to, mammals such as bovine, canine, caprine, cervine, equine, feline, human, ovine, porcine and primates. The subject may be a companion animal. Preferably, the subject is a human. In various embodiments, the subject may have, or be suspected of or at risk of, a condition associated with elevated insulin levels and/or reduced insulin sensitivity.

A condition associated with elevated insulin levels and/or reduced insulin sensitivity includes insulin resistance, prediabetes, type 2 diabetes, metabolic syndrome, gestational diabetes or cardiovascular disease.

The subject may be at risk of developing, have a predisposition for or be suffering from insulin resistance.

The subject may be an insulin resistant subject at risk of developing, have a predisposition for or be suffering from Type II Diabetes.

### USE

The present invention also provides the use of an omega-3 PUFA or a nutritional composition comprising an omega-3 PUFA as an active agent for reducing insulin levels in a subject having a PPARγ2 X12A genetic variant, where X is proline or alanine.

The nutritional composition may be any nutritional composition as described herein.

In another aspect, the present invention provides an omega-3 PUFA or a nutritional composition comprising an omega-3 PUFA as an active agent for use in reducing insulin levels in a subject previously identified to have a PPARγ2 X12A genetic variant, where X is proline or alanine.

The subject may be suffering from a condition referred to herein which is associated with elevated insulin levels and/or reduced insulin sensitivity.

In a further aspect, the present invention provides an omega-3 PUFA or a nutritional composition comprising an omega-3 PUFA as an active agent for use in preventing or treating a condition associated with reduced insulin sensitivity in a subject previously identified to have a PPARγ2 X12A genetic variant, where X is proline or alanine.

### METHOD

In one aspect the present invention relates to a method for reducing insulin levels in a subject comprising administering an omega-3 PUFA or a nutritional composition comprising an omega-3 PUFA as an active agent to the subject; wherein the subject has been identified as having a PPARγ2 X12A genetic variant, where X is proline or alanine.

The subject may be suffering from a condition referred to herein which is associated with elevated insulin levels and/or reduced insulin sensitivity.

In one aspect the present invention relates to a method for preventing or treating a condition associated with reduced insulin sensitivity in a subject comprising administering an omega-3 PUFA or a nutritional composition comprising an omega-3 PUFA as an active agent to the subject; wherein the subject has been identified as having a PPARγ2 X12A genetic variant, where X is proline or alanine.

The present methods comprise administering an effective amount of an omega-3 PUFA (for example as a nutritional composition comprising an omega-3 PUFA) to the subject. An effective amount refers to an amount which is capable of, for example, reducing insulin levels in the subject and/or preventing or treating a condition associated with reduced insulin sensitivity in the subject.

The effective amount may differ depending on the weight, age or sex of the subject.

The present methods may comprise the steps of: i) determining whether the subject has a PPARγ2 X12A genetic variant; and ii) administering an omega-3 PUFA or a nutritional composition comprising an omega-3 PUFA as an active agent to a subject identified has having a PPARγ2 X12A genetic variant, where X is proline or alanine.

Determining the presence of a genetic variant may be performed using standard methods in the art such as those described herein.

In a further aspect the invention provides a method for determining whether a subject is susceptible to benefit from the consumption of PUFA comprising the steps of: a) determining whether the subject is positive for a PPARγ2 X12A genetic variant and b) determining if the subject is susceptible to benefit from the consumption of PUFA depending of the carried genetic variant, wherein such benefit is related to the reduction of insulin sensitivity.

Those skilled in the art will understand that they can freely combine all features of the present invention described herein, without departing from the scope of the invention as disclosed.

Various preferred features and embodiments of the present invention will now be described by way of non-limiting examples.

### EXAMPLES

### Example 1 - Impact of omega-3 PUFA supplementation on fasting insulin levels

The bioefficacy of omega-3 PUFA (provided by intake of fish oil supplements) on fasting insulin levels was assessed.

The present inventors determined that individuals carrying a PPARγ2 Pro12Ala (PPARγ2 X12A) variant allele had a significantly greater reduction in fasting insulin levels compared to individuals homozygous for PPARγ2 Pro12 following a six week omega-3 PUFA supplementation (see Figure 1 and Table 1).

In particular, the results of the present study demonstrated that a six week supplementation with omega-3 PUFA (performed as outlined in the Methods) resulted in a reduction of fasting insulin levels of approximately 20% in individuals carrying the PPARγ2 Pro12Ala variant (PPARγ2 X12A subgroup) compared to approximately 2.5% in individuals homozygous for PPARγ Pro12 (PPARγ2 P12P subgroup).

**Table 1 - Fasting serum insulin response to omega-3 PUFA supplementation**

| Insulin (mIU/L) | | Baseline | Week 6 | Change (Week 6-Baseline) | %Change ((Week 6/Baseline)-1)*100 |
|---|---|---|---|---|---|
| | **N** | **38** | **37** | **37** | **37** |
| **PPARγ2 X12A** | **Mean** | **10.550 (± 12.900)** | **8.455 (± 9.411)** | **-2.357 (± 8.382)** | **-19.826** |
| | **Median** | **6.125** | **5.875** | **-0.744** | **-10.227** |
| | | | | | |
| | N | 153 | 152 | 152 | 152 |
| PPARγ2 P12P | Mean | 6.994 (± 10.816) | 6.890 (± 6.627) | -0.106 (± 10.681) | -2.474 |
| | Median | 5.666 | 5.580 | -0.211 | -4.216 |
| | | | | | |
| ALL | N | 191 | 189 | 189 | 189 |
| | Mean | 7.701 (± 11.314) | 7.197 (± 7.254) | -0.547 (± 10.290) | -6.144 |
| | Median | 5.690 | 5.633 | -0.302 | -5.434 |

In addition, there was a significant difference in changes in adiponectin levels between the PPARγ2 X12A subgroup and the PPARγ2 P12P subgroup at a threshold of 10% (p=0.063). In particular, there was an estimated median difference in change of 0.37 ug/ml when excluding outliers.

### Materials and methods

### Study participants

191 healthy male and female subjects between 18 to 40 years of ages, with a BMI between 18.5 and 30, non-smokers, with sedentary to moderate physical activity and no consumption of dietary supplements or any medication that can affect the study outcome.

### Omega-3 PUFA dosage

The participants' diets were supplemented with omega-3 PUFA at a dose of 2700 mg day, as provided by three fish oil capsules (GNC Preventive Nutrition^{®} Triple Strength Fish Oil), to be orally administered each day. Each capsule contains 647 mg eicosapentaenoic acid (EPA) and 253 mg docosahexaenoic acid (DHA).

### Study procedure

Participants visited the clinical setting on three occasions, three weeks apart. After a screening interview, potential participants were invited to the study site for their first visit during which they provided informed consent. After verification of the inclusion/exclusion criteria, enrolled participants undertook the following tests: clinical history, food frequency questionnaire, validated physical activity questionnaire, blood pressure measurement, anthropometric measurements (weight, height and waist circumference), body composition by bioelectric impedance using the InBody720 equipment (Biospace Co, Ltd.). A 12.5 ml blood sample was drawn under fasting conditions (> 8 hrs) for measurements of HbA1c %, glucose, insulin, triglycerides, total cholesterol, lipoprotein fractions, adiponectin and CRP in plasma and separation of PBMC for RNA and DNA isolation. 2.5 ml EDTA-blood was drawn for determination of fatty acid in the plasma membrane of erythrocytes and 10 ml for PBMC transcriptomics. Participants were also provided with the supplements for the next three weeks.

During a second visit, the results of HbA1c %, glucose, lipids, insulin and body composition were discussed by a nutritionist with the subjects. The subjects returned a side effects journal, filled out a 24 hour food recall questionnaire and provided with the supplements for the last three weeks.

During the second visit, the participants discussed the results of the HbA1c%, glucose, lipids, insulin and body composition tests with a nutritionist. Participants also returned return the side effects journal, filled out a 24 hr food recall questionnaire and were provided with the supplements for the last three weeks. The last visit, after six weeks supplementation, had the same parameters as the first visit, except that the clinical history and SNUT food frequency questionnaire were recorded. The 24 hr food recall questionnaire and physical activity questionnaires were collected on all three visits.

All publications mentioned in the above specification are herein incorporated by reference. Various modifications and variations of the described methods of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

SEQUENCE LISTING

<110> INSTITUTO NACIONAL DE MEDICINA GENÃ\223MICA

<120> Nutritional Composition

<160> 1

<170> BiSSAP 1.2

<210> 1
<211> 505
<212> PRT
<213> Homo sapiens

<400> 1

## Claims

1. A nutritional composition comprising an omega-3 PUFA as an active agent for use in reducing insulin levels in a subject having a peroxisome proliferator-activated receptor-gamma 2 (PPARγ2) X12A genetic variant, where X is proline or alanine.

2. A nutritional composition comprising an omega-3 PUFA as an active agent for use in treating or preventing reduced insulin sensitivity or a condition associated with reduced insulin sensitivity in a subject having a PPARγ2 X12A genetic variant, where X is a proline or alanine.

3. A nutritional composition according for use according to claim 2 wherein the condition associated with reduced insulin sensitivity is insulin resistance, prediabetes, type 2 diabetes, metabolic syndrome, gestational diabetes or cardiovascular disease.

4. A nutritional composition for use according to any preceding claim wherein the subject is administered from about 500 to about 10000 mg omega-3 PUFA per day, preferably from about 1500 to about 3000 mg omega-3 PUFA per day.

5. A nutritional composition for use according to any preceding claim wherein the omega-3 PUFA is eicosapentanoic acid (EPA) and/or docosahexanoic acid (DHA).

6. A nutritional composition for use according to claim 5 wherein the omega-3 PUFA comprises an EPA to DHA ratio of about 0.5:1, to about 5:1 wt/wt; preferably about 2.5:1 wt/wt.

7. A nutritional composition for use according to claim 6 wherein the subject is administered about 500 to 4000 mg EPA and about 500 to 4000 mg DHA per day, preferably about 1000 to 3000 mg EPA and about 500 to 1000 mg DHA per day.

8. A nutritional composition for use according to any preceding claim wherein the composition is in the form of a food stuff.

9. A nutritional composition for use according to claim 8 wherein the composition is in the form of a human food stuff.

10. A nutritional composition for use according to any of claims 1 to 7 wherein the composition is in the form of a complete nutritional product.

11. A nutritional composition for use according to any of claims 1 to 7 wherein the composition is in the form of a capsule, a tablet, a powdered form, a freeze-dried product or an oil-in-water emulsion.

12. A method for reducing insulin levels in a subject comprising administering a nutritional composition comprising an omega-3 PUFA as an active agent to the subject; wherein the subject has been identified as having a PPARγ2 X12A genetic variant, where X is a proline or alanine.

13. A method for reducing insulin levels in a subject comprising the steps of:
i) determining whether the subject has a PPARγ2 X12A genetic variant; and
ii) administering a nutritional composition comprising an omega-3 PUFA as an active agent to a subject identified has having a PPARγ2 X12A genetic variant; where X is a proline or alanine.

14. A method according to 12 or 13 for use in treating or preventing reduced insulin sensitivity or a disease associated therewith, insulin resistance, prediabetes, type 2 diabetes, metabolic syndrome, gestational diabetes or cardiovascular disease.

15. Use of an omega-3 PUFA in the preparation of a nutritional composition for reducing insulin levels in a subject or preventing or treating a condition associated with reduced insulin sensitivity in a subject, wherein the subject has a PPARγ2 X12A genetic variant; where X is a proline or alanine.
